# EUROPEAN PATENT APPLICATION

(11) **EP 4 129 403 A1**
(43) Date of publication of application: **08.02.2023**
(21) Application number: 21781960.6
(22) Date of filing: 18.03.2021
(51) Int. Cl.: A61P 3/00, A61P 43/00, A61K 36/064, A23L 31/10, A23L 33/10, A23L 33/14, A23L 33/175, A61K 47/36, A61K 31/132, A61K 31/198

(54) **BODY FAT REDUCING AGENT**

(30) Priority: 03.04.2020 JP 2020067731
(71) Applicant: MITSUBISHI GAS CHEMICAL COMPANY, INC., Chiyoda-ku Tokyo 100-8324 (JP)
(72) Inventor: NAKAMURA, Kenji, Niigata-shi, Niigata 950-3112 (JP); FURUTANI, Masayuki, Niigata-shi, Niigata 950-3121 (JP); TACHIKAWA, Tomokazu, Tokyo 100-8324 (JP)
(74) Representative: Vossius & Partner Patentanwälte Rechtsanwälte mbB
(86) International application number: PCT/JP2021/011077
(87) International publication number: WO 2021/200214

(57) **Abstract**

A body fat reducer containing a polyamine and a branched-chain amino acid as active ingredients.

## Description

### Technical Field

The present invention relates to a body fat reducer.

### Background Art

With the recent changes in food culture, the fat intake of Japanese people is increasing. Excessively ingested lipids are accumulated in the body in the form of fat, which causes obesity and the like. Obesity is known to be associated with various health disorders and causes lifestyle-related diseases such as metabolic syndrome and dyslipidemia.

In view of such circumstances, various oral preparations have been reported in recent years for the purpose of reducing body fat for obesity. For example, lactoferrin (Patent Literature 1), which suppresses the accumulation of fat and reduces visceral fat by decomposing the accumulated fat, and a tea extract (Patent Literature 2) having an action of promoting the burning of body fat have been reported. If such a body fat reducer can be provided, it is considered to be widely used for preventing or improving obesity and metabolic syndrome, and slimming.

### Citation List

### Patent Literature

Patent Literature 1: Japanese Patent Laid-Open No.2011-001333
Patent Literature 2: Japanese Patent Laid-Open No.2006-117687

### Summary of Invention

### Technical Problem

The present invention has been devised in view of the problems described above, and an object thereof is to provide a novel body fat reducer.

### Solution to Problem

As a result of diligent studies in order to solve the above problems, the inventors have found that the aforementioned problems can be solved by using a polyamine and a branched-chain amino acid in combination, thereby accomplishing the present invention.

That is, the present invention is as follows.
[1] A body fat reducer comprising a polyamine and a branched-chain amino acid as active ingredients.
[2] The body fat reducer according to [1], wherein the polyamine is one or more selected from the group consisting of putrescine, spermidine, and spermine.
[3] The body fat reducer according to [2], comprising 1 mg/g or more of spermidine per 1 g of the body fat reducer.
[4] The body fat reducer according to any one of [1] to [3], wherein the polyamine is derived from yeast.
[5] The body fat reducer according to [4], comprising a polyamine-containing yeast as the polyamine.
[6] The body fat reducer according to [5], comprising 200 to 800 mg of the polyamine-containing yeast in terms of dry mass per 1 g of the body fat reducer.
[7] The body fat reducer according to [5] or [6], comprising 1 mg/g or more of spermidine in terms of dry mass at a moisture content of 6.0% or less.
[8] The body fat reducer according to any one of [5] to [7], further comprising a crystalline polysaccharide.
[9] The body fat reducer according to [8], wherein the mass ratio between the yeast and the crystalline polysaccharide is 1:0.1 to 1.
[10] The body fat reducer according to any one of [4] to [9], wherein the yeast is a yeast belonging to the genus Saccharomyces.
[11] The body fat reducer according to any one of [1] to [10], wherein the branched-chain amino acid is one or more selected from the group consisting of L-valine, L-leucine, and L-isoleucine.
[12] The body fat reducer according to [11], comprising 20 to 30 parts by mass of L-valine, 40 to 60 parts by mass of L-leucine, and 20 to 30 parts by mass of L-isoleucine, based on 100 parts by mass in total of L-valine, L-leucine, and L-isoleucine.
[13] The body fat reducer according to any one of [1] to [12], comprising 200 to 800 mg of the branched-chain amino acid in terms of dry mass per 1 g of the body fat reducer.
[14] A composition comprising the body fat reducer according to any one of [1] to [13].
[15] The composition according to [14], being in the form of a food or drink.

### Advantageous Effect of Invention

The present invention can provide a novel body fat reducer.

### Description of Embodiments

Hereinafter, the embodiments of the present invention (which will be hereinafter referred to as "the present embodiment(s)") will be described in detail, but the present invention is not limited to these embodiments, and various modifications are possible within the range that does not deviate from the gist.

### [Body fat reducer]

The body fat reducer of the present embodiments contains a polyamine and a branched-chain amino acid as active ingredients, and may contain another additive such as a crystalline polysaccharide, as required.

A polyamine, which is a linear in vivo aliphatic hydrocarbon having two or more primary amino groups, has a high effect on cell activation, and foods containing it have been provided. Known physiological actions of polyamine include cell proliferation action, cell differentiation-promoting action, immune essential factor, antiallergic action, protein synthesis-promoting action, structural stabilization by interaction with nucleic acid, and enzyme activity-regulating action. Recently, it has been found that a novel body fat reducer can be provided by using such a polyamine in combination with a branched-chain amino acid. Hereinafter, each component will be described in detail.

### (Polyamine)

A polyamine is a general term for linear aliphatic hydrocarbons in which multiple amino groups are bonded. Examples thereof include putrescine, spermidine, spermine, 1,3-diamino propane, cadaverine, caldin, homospermidine, aminopropylcadaverine, theremin, thermospermine, canavalmine, aminopentylnorspermidine, N,N-bis(aminopropyl)cadaverine, homospermine, caldopentamine, homocaldopentamine, caldohexamine, and homocaldohexamine spermidine. Among these, putrescine, spermidine, and spermine are preferable. Such a polyamine has a high cell activation ability and a high utilization rate in the body, and is excellent in terms of functionality. In particular, spermidine has a higher acceptable daily intake than spermine and is preferred for higher intakes. Further, use of such a polyamine tends to further improve the body fat-reducing effect by combination use with the branched-chain amino acid.

The content of polyamine per 1 g of the body fat reducer is preferably 1 mg/g or more, more preferably 1 to 10 mg/g, further preferably 1 to 5 mg/g. The content of polyamine falling within such a range tends to further improve the body fat-reducing effect. The content of polyamine is not limited to the aforementioned ranges, and the polyamine can be used within the acceptable daily intake range of the specific compound such as spermidine.

The method for producing a polyamine is not particularly limited. For example, known methods include a preparation method involving treating yeast bacterial cells or yeast culture solutions under acidic conditions, an extraction method involving subjecting plant materials under acidic conditions, a method of producing polyamine extracts by adding a solution of salt such as sodium chloride, magnesium chloride, and calcium chloride to plant materials, or a method for obtaining a polyamine-containing yeast as described in International Publication No. WO 2018/168525.

Among these, the polyamine to be used in the present embodiments is preferably derived from yeast, and it is more preferable to use a polyamine-containing yeast as the polyamine. As the yeast, yeast belonging to the genus Saccharomyces, preferably Saccharomyces cerevisiae, can be used. Use of such a polyamine tends to further improve the body fat-reducing effect. In this case, the polyamine to be contained can be 1.5 mg or more, preferably 2.0 mg or more, more preferably 2.5 mg or more, per 1 g (dry mass) of yeast.

The dry yeast containing a polyamine can be obtained by removing bacterial cells from a medium containing yeast by filtration or centrifugation, followed by drying. In order to remove the medium components, washing may be performed. For drying the yeast, spray drying, vacuum drying, freeze drying, and the like can be used, and those skilled in the art can appropriately select a drying method according to the purpose.

The "dry mass" in the present embodiments means the mass when the moisture content is 6.0% or less, unless otherwise specified.

The polyamine to be contained in the body fat reducer may be added in the form of a compound of the polyamine or may be added in the form of a polyamine-containing yeast, as mentioned above. Among these, addition in the form of a polyamine-containing yeast is preferable.

In the case of adding a polyamine in the form of a polyamine-containing yeast to the body fat reducer, the dry mass thereof is preferably 200 to 800 mg, more preferably 300 to 700 mg, further preferably 400 to 600 mg, per 1 g of the body fat reducer. The dry mass of the polyamine-containing yeast falling within such a range tends to further improve the body fat-reducing effect. The content of the polyamine is not limited to the aforementioned ranges, and it can be used within the acceptable daily intake range of the specific compound such as spermidine.

In the case of addition in the form of a polyamine-containing yeast, the content of spermidine is preferably 0.8 mg/g or more, more preferably 0.9 mg/g or more, further preferably 1 mg/g or more, in terms of dry mass at a moisture content of 6.0% or less, per 1 g of the body fat reducer. The dry mass of polyamine-containing yeast falling within such a range tends to further improve the body fat-reducing effect. The upper limit of the content of spermidine in the case of addition in the form of a polyamine-containing yeast is not particularly limited but is preferably 10 mg/g or less, more preferably 5 mg/g or less.

### (Branched-chain amino acid)

A branched-chain amino acid (BCAA) generally refers to one or more selected from the group consisting of L-valine, L-leucine, and L-isoleucine among nine essential amino acids that cannot be synthesized in the human body and is an important nutrient serving as an energy source for muscles. Branched-chain amino acids are abundantly contained in meat, fish, dairy products, eggs, etc., but since they are ingested as proteins from foods, it takes several hours before they are decomposed into branched-chain amino acids and absorbed. Therefore, in order to efficiently absorb branched-chain amino acids, it is effective to ingest the branched chain amino acids as they are.

The body fat reducer of the present embodiments preferably contains L-valine, L-leucine, and L-isoleucine as branched-chain amino acids, respectively. In this case, regarding the mass ratio between L-valine, L-leucine, and L-isoleucine, 20 to 30 parts by mass of L-valine, 40 to 60 parts by mass of L-leucine, and 20 to 30 parts by mass of L-isoleucine, based on 100 parts by mass in total of L-valine, L-leucine, and L-isoleucine are preferably contained. The branched-chain amino acids contained at such a mass ratio tend to further improve the body fat-reducing effect.

The content of branched-chain amino acids in the body fat reducer of the present embodiments is preferably 200 to 800 mg, more preferably 300 to 700 mg, further preferably 400 to 600 mg, in terms of dry mass, per 1 g of the body fat reducer. The content of branched-chain amino acids falling within such a range tends to further improve the body fat-reducing effect.

The method for producing branched-chain amino acids is not specifically limited, and they can be produced by the fermentation method, the synthesis method, the purification method, or the like. Commercially available products also can be used. As the branched-chain amino acids used in the present invention, common commercially available branched-chain amino acids can be used.

### (Crystalline polysaccharide)

The body fat reducer of the present embodiments may contain a crystalline polysaccharide. Containing a crystalline polysaccharide can impart fluidity or deodorization to the body fat reducer and also can impart stability to the polyamine or polyamine-containing yeast. Further, it is also possible to suppress fat absorption and contribute to the body fat-reducing effect.

The crystalline polysaccharide is not particularly limited, and examples thereof include polysaccharides that exist as naturally crystalline solids such as starch, dextrin, glycogen, and cellulose. Among these, cyclodextrin, which is a dextrin having a cyclic structure, can be suitably used as a crystalline polysaccharide. There are alpha, beta, and gamma types of cyclodextrin, all of which can be used as crystalline polysaccharides. Among these, alpha cyclodextrin and gamma cyclodextrin are preferable. Other dextrins such as resistant dextrins can also be used for the composition of the present invention.

It is possible to stabilize the yeast to be easily handled by combining a polyamine-rich yeast with a crystalline polysaccharide. The yeast and the crystalline polysaccharide may be mixed by any method of dry mixing and wet mixing. In the case of wet mixing, the saccharide after being sterilized and mixed with the yeast can be dried. Alternatively, the yeast may be mixed as undried yeast and then dried, instead of being mixed as dried yeast. Further, yeast after being cultured may be collected/concentrated by centrifugation, a crystalline polysaccharide may be added thereto, and then the yeast and the crystalline polysaccharide may be mixed by spray drying.

The content of crystalline polysaccharide is preferably 30 to 500 mg, more preferably 40 to 400 mg, further preferably 50 to 300 mg, per 1 g of the body fat reducer. The content of crystalline polysaccharide falling within such a range tends to further improve the body fat-reducing effect.

In the case of using a polyamine-containing yeast, the mass ratio between the dry mass of the polyamine-containing yeast and the crystalline polysaccharide is preferably 1:0.1 to 3, more preferably 1:0.1 to 2, further preferably 1:0.1 to 1. The mass ratio between the dry mass of the polyamine-containing yeast and the crystalline polysaccharide falling within such a range not only further improves the stability of the polyamine-containing yeast, but also can ensure the content of polyamine, especially spermidine in the body fat reducer to a certain extent, so that the effect of the body fat reducer can be obtained with an appropriate intake.

### [Composition]

The composition of the present embodiments contains the body fat reducer described above. The application thereof is not particularly limited, and it can be used, for example, for not only various general foods or drinks, but also foods or drinks, pharmaceutical products, non-medicinal products, and cosmetics that are labeled with functionality so that they can be ingested by individuals who needs nutrient enrichment, especially, individuals who needs cell activity.

Such foods or drinks are not particularly limited, and examples thereof include specified health foods or drinks, nutritional functional foods or drinks, functional epidermal foods or drinks, health functional foods or drinks, special purpose foods or drinks, nutritional supplements, health supplements, supplements, beauty foods or drinks, and other health foods or drinks.

### Examples

Hereinafter, the present invention will be described more specifically with reference to examples and comparative examples. The present invention is not limited by the following examples at all.

### (Preparation of test foods)

### (Capsules A)

A BCAA agent as a branched-chain amino acid was prepared at a mass ratio of L-valine:L-leucine:L-isoleucine of 1:2:1. 123 mg of Elion SP (R), available from MITSUBISHI GAS CHEMICAL COMPANY, INC., which is a yeast food containing spermidine, and 112 mg of the BCAA agent prepared above were mixed and put into hard capsules No. 2, to prepare capsules A. The dry mass of the spermidine-containing yeast in Elion SP (R) was adjusted to 100 mg. 3 mg of spermidine was contained per 1 g of the dry mass of the spermidine-containing yeast.

### (Capsules B)

123 mg of corn starch and 112 mg of the BCAA agent prepared above were mixed and put into hard capsules No. 2, to prepare capsules B for placebo.

### (Capsules C)

235 mg of corn starch was put into hard capsules No. 2, to prepare capsules C for placebo.

### (Grouping of subjects and daily intake)

Subjects satisfying the following conditions were selected and subjected to the following tests.
· Healthy men over 50 years old (50s, 60s, and 70s) who have not been treated for illness
· Those with a BMI value of 18.5 or more and less than 30
· Those who have not taken supplements (BCAAs, proteins, etc.) that may affect muscles within one month before the start of the test
· Those who have not taken Natto, which contains a lot of polyamines, more than twice a week

A pre-screening test (before ingestion) was performed on the aforementioned subjects to determine suitability. Then, the subjects were classified into the following Groups I, II, and III based on the daily capsule intake.
Group I: Group taking 3 capsules A and 6 capsules B per day, with a yeast intake of 300 mg/day and a BCAA agent intake of 1008 mg/day
Group II: Group taking 9 capsules B per day, with a yeast intake of 0 mg/day and a BCAA agent intake of 1008 mg/day
Group III: Group taking 9 capsules C per day, with a yeast intake of 0 mg/day and a BCAA agent intake of 0 mg/day

BCAA agents have already been commercially available from many manufacturers, and most products recommend an intake of approximately 2000 mg or more per day. However, in order to determine the effect of polyamine, the intake of the BCAA agent in this evaluation was set to 1008 mg (about 1/2 of the general recommended intake).

### (Test method)

Human oral clinical trials were conducted in a double-blind study on subjects in Groups I, II, and III. Specifically, during the test period, each subject took a total of 9 capsules within 30 minutes after each breakfast, and this test was conducted for 8 weeks. During the test period, the subject was asked to keep a daily step count, exercise, and dietary records as a guideline for exercise indicators. After that, the subject was inspected for the same items as in the pre-examination.

### (Inspection items)

Before and after the test, the subjects were inspected for body fat percentage, lean body mass, and BMI with a body composition analyzer as a physiological test in addition to a blood test. The definition of each term is shown below (see the instruction manual of TANITA CORPORATION). The body composition analyzer used is MC-980A-N plus, available from TANITA CORPORATION. This body composition analyzer is a body composition analyzer using the bioelectrical impedance method.
Body fat percentage: Ratio of fat mass to body weight
Lean body mass: Body weight minus fat
BMI: Body weight(kg)/height²(m)

### (Analysis)

The difference in each item before and after the test measured as described above was evaluated by the Wilcoxon rank sum test among the nonparametric tests. In this evaluation, when the p-value is p < 0.05 (*), it was determined as being statistically significant, and when the p-value is p < 0.01 (**), it was determined as being highly significant.

The correlation coefficient (r) between the intake of Elion SP (R) and the difference before and after the test was determined in Groups I, II, and III. In this evaluation, when the r value is 0.4 ≤ | r | ≤ 0.7, it is regarded as "correlated (*)", and when the r value is 0.7 < | r | < 1.0, it is "highly correlated (**)".

### (Results 1)

Table 1 shows the results when the aforementioned analysis was performed on the subjects belonging to each of Group I and Group II. In a nonparametric test, no significant difference was found between the intake of Elion SP (R) and the items related to the body fat reduction in Group I. However, in correlation coefficient, correlations were found between the intake of Elion SP (R) and the body fat percentage, the lean body mass, and the BMI.

**[Table 1]**

| | n number | Average number of steps | Body fat percentage | Lean body mass | BMI |
|---|---|---|---|---|---|
| GROUP II (Average value) | 7 | 9315 | -0.286 | 0.257 | 0.036 |
| GROUP I (Average value) | 10 | 8303 | -1.520 | 1.000 | -0.020 |
| GROUP I (p-value) | | | p≥0.05 | p≥0.05 | p≥0.05 |
| Correlation coefficient (r) | - | - | 0.891** | 0.533* | 0.994** |

### (Results 2)

Table 2 shows the results of performing statistical calculations again based on Group III, with the difference before and after the test taken as 0. As a result, in a nonparametric test, significant differences were found between the intake of Elion SP (R), the body fat percentage, and the lean body mass in Group I, and no significant difference was found between the intake of Elion SP (R) and the items related to the body fat reduction in Group II.

**[Table 2]**

| | n number | Body fat percentage | Lean body mass | BMI |
|---|---|---|---|---|
| GROUP III | 10 | - | - | - |
| GROUP II | 7 | p≥0.05 | p≥0.05 | p≥0.05 |
| GROUP I | 10 | p<0.01 ** | p<0.01 ** | p≥0.05 |

### (Results 3)

Table 3 shows the results when the subjects belonging to each of Group I and Group II were further divided into "exercisers" and "non-exercisers", and the aforementioned analysis was performed on "exercisers".

The "exercisers" were defined as those with an average number of steps per day equal to or higher than the literature value of the average number of steps in each age group, including the second quartile borders when divided by quartiles. The literature was cited from "Table 61 of the FY2017 National Nutrition Survey" published on the website of the Ministry of Health, Labour and Welfare.

From these results, significant differences were found between body fat percentage and lean body mass in Group I, and correlations were also found between body fat percentage, lean body mass, and BMI in the correlation coefficient.

**[Table 3]**

| | n number | Average number of steps | Body fat percentage | Lean body mass | BMI |
|---|---|---|---|---|---|
| GROUP II (Average value) | 4 | 11181 | 0.550 | -0.400 | 0.038 |
| GROUP I (Average value) | 5 | 10597 | -2.160 | 1.200 | -0.156 |
| GROUP I (p-value) | | | p<0.05* | p<0.05* | p≥0.05 |
| Correlation coefficient (r) | - | - | 0.828** | 0.554* | 0.995** |

### Industrial Applicability

The present invention has industrial applicability in that it provides a novel body fat reducer.

## Claims

1. A body fat reducer comprising:
a polyamine and a branched-chain amino acid as active ingredients.

2. The body fat reducer according to claim 1, wherein
the polyamine is one or more selected from the group consisting of putrescine, spermidine, and spermine.

3. The body fat reducer according to claim 2, comprising:
1 mg/g or more of spermidine per 1 g of the body fat reducer.

4. The body fat reducer according to any one of claims 1 to 3, wherein
the polyamine is derived from yeast.

5. The body fat reducer according to claim 4, comprising:
a polyamine-containing yeast as the polyamine.

6. The body fat reducer according to claim 5, comprising:
200 to 800 mg of the polyamine-containing yeast in terms of dry mass per 1 g of the body fat reducer.

7. The body fat reducer according to claim 5 or 6, comprising:
1 mg/g or more of spermidine in terms of dry mass at a moisture content of 6.0% or less.

8. The body fat reducer according to any one of claims 5 to 7, further comprising:
a crystalline polysaccharide.

9. The body fat reducer according to claim 8, wherein
the mass ratio between the yeast and the crystalline polysaccharide is 1:0.1 to 1.

10. The body fat reducer according to any one of claims 4 to 9, wherein
the yeast is a yeast belonging to the genus Saccharomyces.

11. The body fat reducer according to any one of claims 1 to 10, wherein
the branched-chain amino acid is one or more selected from the group consisting of L-valine, L-leucine, and L-isoleucine.

12. The body fat reducer according to claim 11, comprising:
20 to 30 parts by mass of L-valine, 40 to 60 parts by mass of L-leucine, and 20 to 30 parts by mass of L-isoleucine, based on 100 parts by mass in total of L-valine, L-leucine, and L-isoleucine.

13. The body fat reducer according to any one of claims 1 to 12, comprising:
200 to 800 mg of the branched-chain amino acid in terms of dry mass per 1 g of the body fat reducer.

14. A composition comprising:
the body fat reducer according to any one of claims 1 to 13.

15. The composition according to claim 14, being in the form of a food or drink.
